(19) Europäisches Patentamt
European Patent Office
Office européen des brevets

(11) Publication number: 0 194 793
A2

(12) # EUROPEAN PATENT APPLICATION

(21) Application number: 86301503.8

(22) Date of filing: 04.03.86

(51) Int. Cl.[4]: C 07 H 17/08
A 61 K 31/70

(30) Priority: 06.03.85 GB 8505786

(43) Date of publication of application:
17.09.86 Bulletin 86/38

(84) Designated Contracting States:
AT BE CH DE FR GB IT LI NL SE

(71) Applicant: Omura, Satoshi
12-7 Seta 5-chome
Setagaya-Ku Tokyo(JP)

(72) Inventor: Omura, Satoshi
12-7, Seta 5-Chome Setagaya-ku
Tokyo(JP)

(72) Inventor: Tsuzuki, Kazuo
18-22 Nishiazabu 4-Chome Minato-ku
Tokyo(JP)

(74) Representative: Hudson, Christopher Mark et al,
Erl Wood Manor
Windlesham Surrey GU20 6PH(GB)

(54) 9-Acyl tylosin derivatives.

(57) 9-0-Acyltylosin derivatives of formula

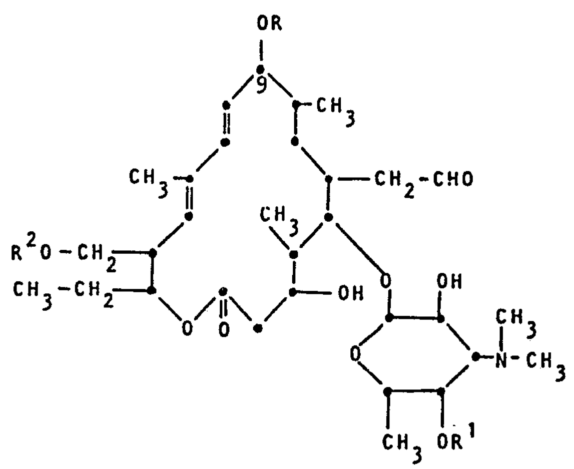

(I)

where
R is a $C_{2-7}$ alkanoyl or benzoyl group optionally substituted by $C_{1-4}$ alkyl, $C_{1-4}$ alkoxy, nitro or halo;

where
$R^1$ is hydrogen or mycarosyl; and
$R^2$ is mycinosyl;
or a pharmaceutically-acceptable salt thereof; are effective antibacterial agents.

EP 0 194 793 A2

# 9-ACYL TYLOSIN DERIVATIVES

This invention relates to macrolide antibiotics, in particular to novel compounds similar to the well-known therapeutic agent tylosin (see, for example, Tetrahedron Letters, 2339 (1970) and U.S. Patent Specification No. 3,178,341) but in which the 9-position of the macrolide system is acylated.

Despite tylosin's great value, there exists a continuing need to develop new antibiotics, both in view of the possibility of providing more active derivatives, possibly having a broader spectrum of activity, and also in view of the known propensity of microorganisms to develop resistance. Unfortunately, chemical modification of tylosin-like macrolides has proven to be extremely difficult. Indeed, in the majority of cases, research workers, intent on finding new derivatives of this type, have been forced to search for new microorganisms, in the hope that their cultivation would fortuitously yield related compounds of interest.

Surprisingly, the Applicant has now discovered that the 9-keto group in tylosin-like structures can be converted, via reduction, into an acyl function, without concomitant disruption of the macrolide system, and further that such derivatives possess significant antibiotic activity.

Thus, in accordance with the invention, novel macrolides of formula (I):

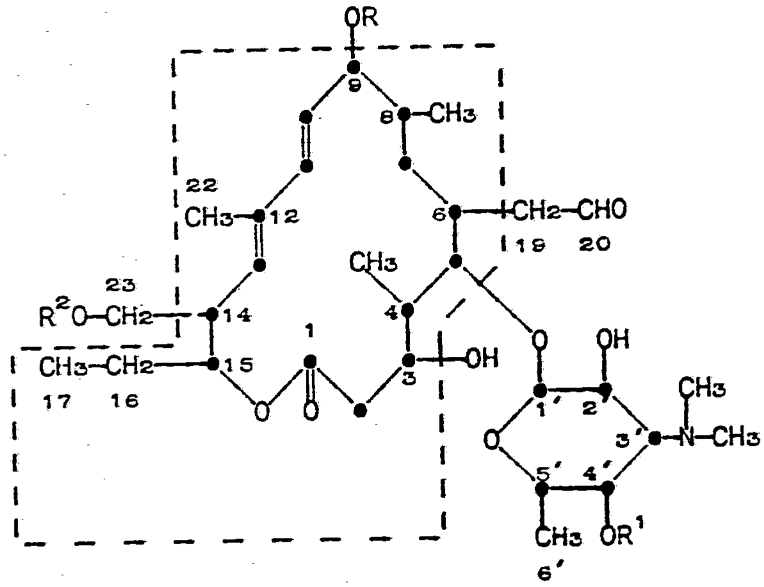

(I)

where R is a $C_{2-7}$ alkanoyl or benzoyl group optionally substituted by $C_{1-4}$ alkyl, $C_{1-4}$ alkoxy, nitro or halo;

where $R^1$ is hydrogen or mycarosyl, i.e.:

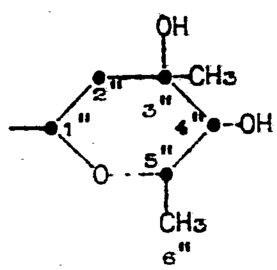

$R^2$ is mycinosyl, i.e.:

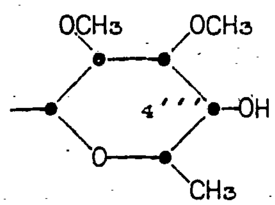

and pharmaceutically-acceptable salts thereof, are effective antibiotics.

Although no stereochemical configuration is indicated in the above structural formula, it is to be understood that the stereochemistry is identical to that of tylosin. The amino sugar is mycaminose.

It is further to be noted that, for the sake of brevity of description in the following specification, the moiety surrounded by the dotted lines in formula (I) above is illustrated merely by the symbol "TYL". It is thus to be understood that the tylosin derivatives according to the present invention represented by the general formula (I) also may be referred to by the following general formula:

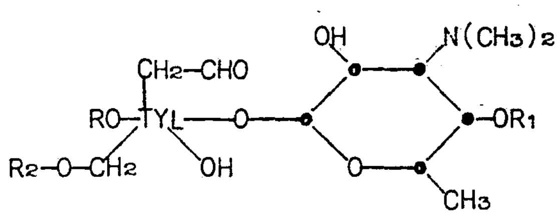

(I)

The tylosin derivatives of the general formula (I) may be produced, in general terms, by acetalizing or thioacetalizing the aldehyde group on the 20-position of tylosin or demycarosyltylosin (Step I); acylating the acetal or thioacetal derivative of tylosin or demycarosyltylosin to protect the hydroxyl group on the 2'-position of the mycaminosyl substituent on the 5-position of the tylosin nucleus (Step II); silylating the 2'-O-acyl derivative of the acetalized or thioacetalized tylosin or demycarosyltylosin to protect all the hydroxyl groups reactive to the acylation on the 9-position of the tylosin nucleus (Step III); reducing the ketone group on the 9-position thereof to give a secondary alcohol group (Step IV); acylating the resulting secondary alcohol group (Step V); hydrolyzing the silyl groups of the silylated 9-O-acylated tylosin or demycarosyltylosin derivative (Step VI); deacylating the acyl group introduced by the step II above (Step VII); and eliminating the acetal or thioacetal group from the 20-position of the tylosin nucleus.

The process for preparing the tylosin derivatives of the general formula (I) will now be described in more detail.

## Reaction Scheme A

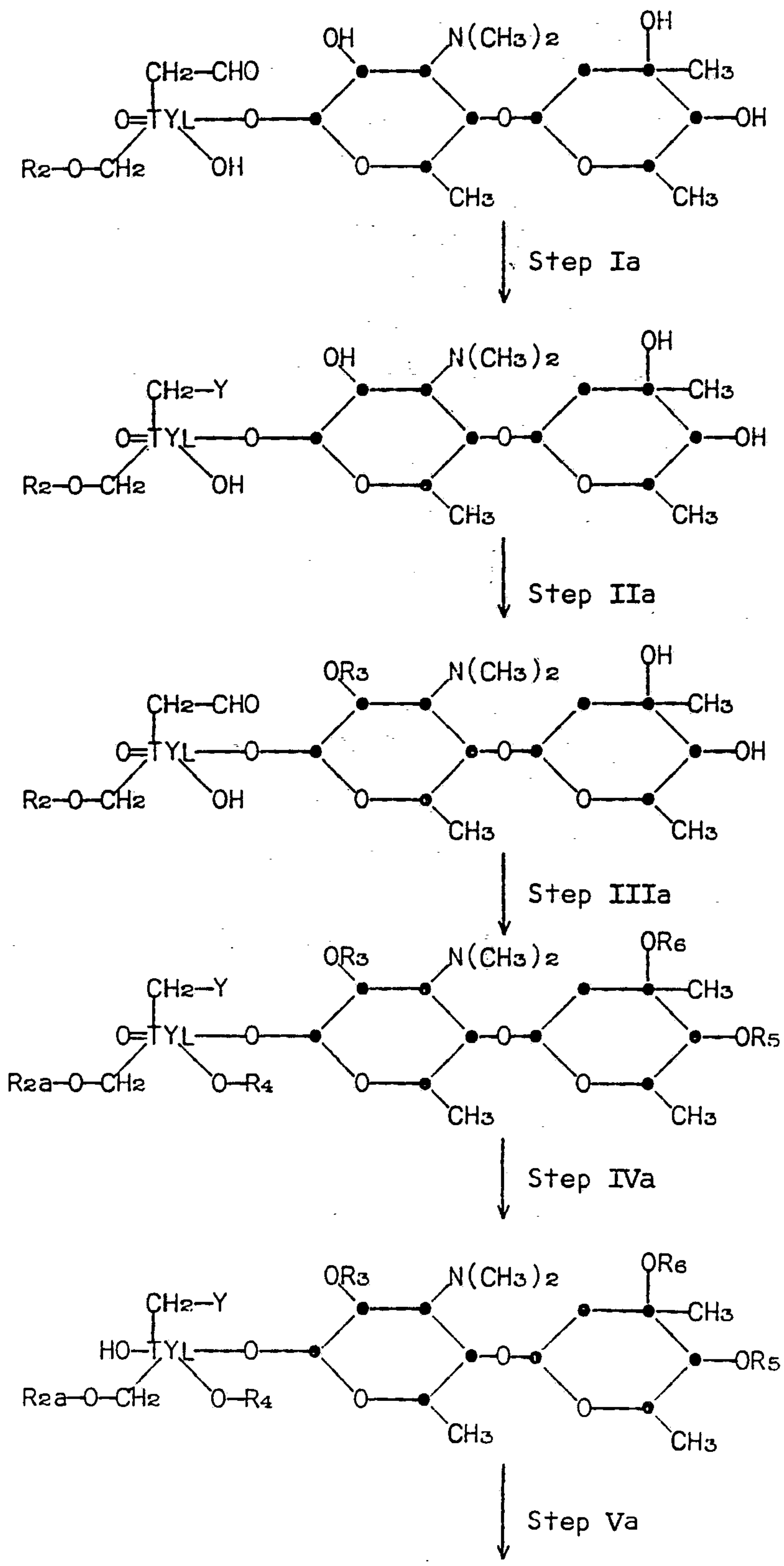


IIa

IIIa

IVa

Va

VIa

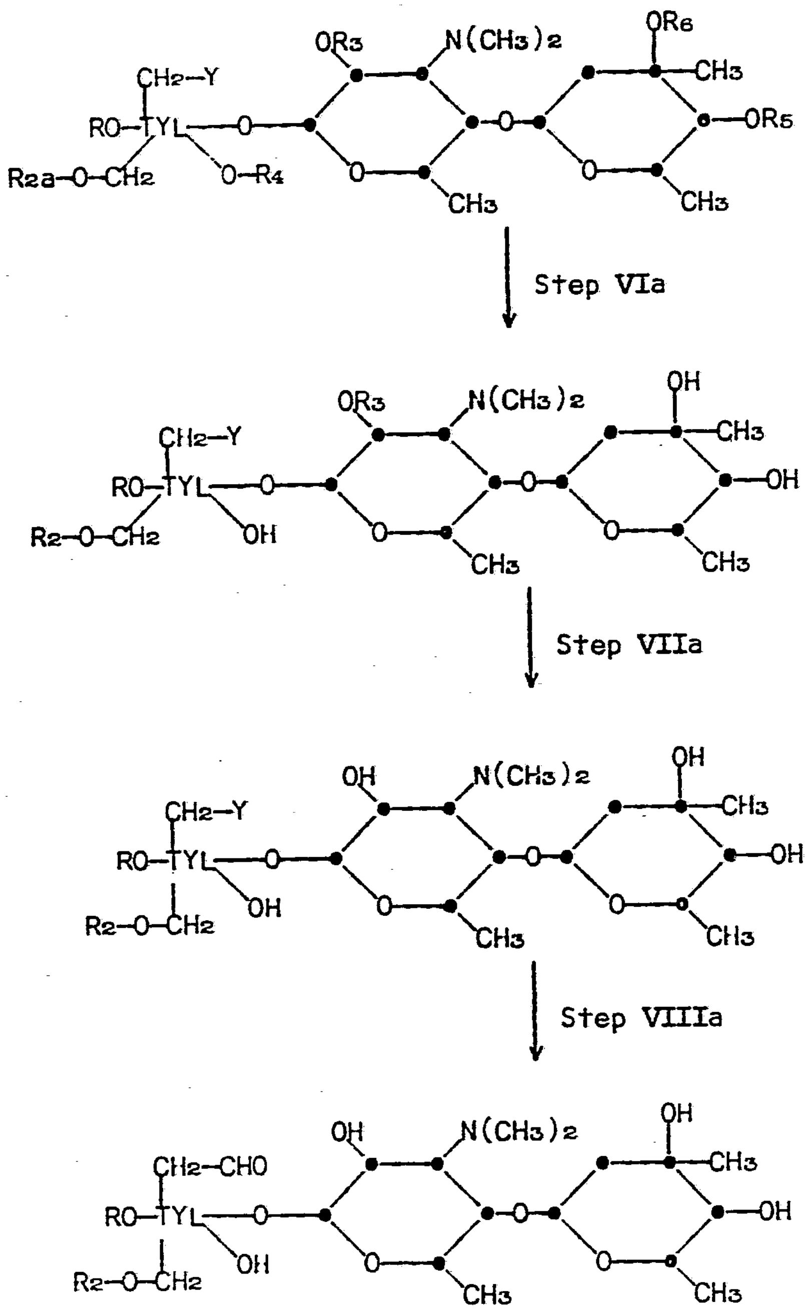


VIIa

VIIIa

IXa

Ia

Reaction Scheme B

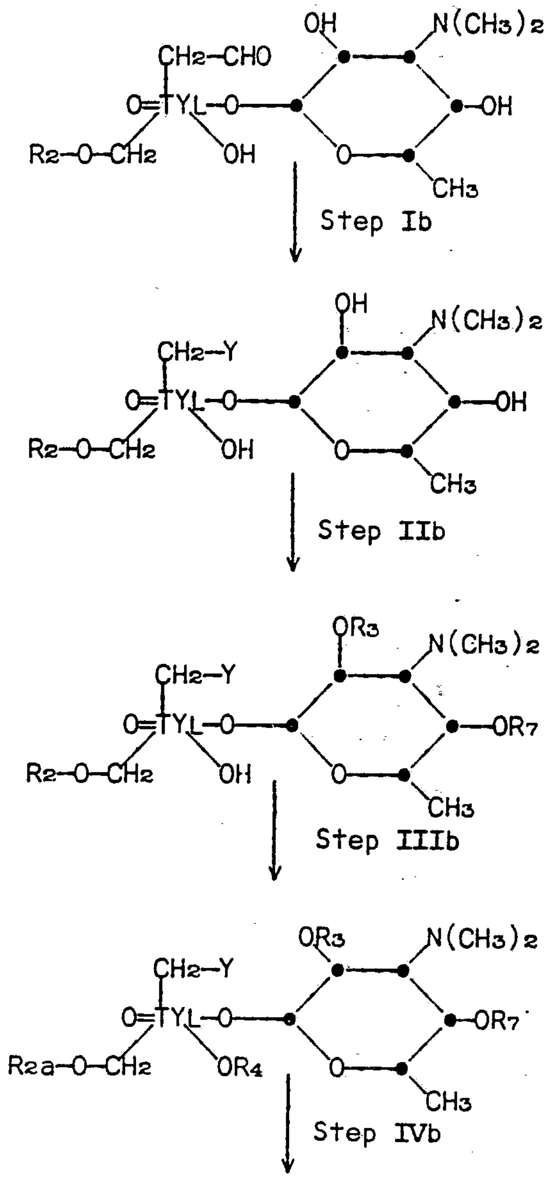


IIb

IIIb

IVb

Vb

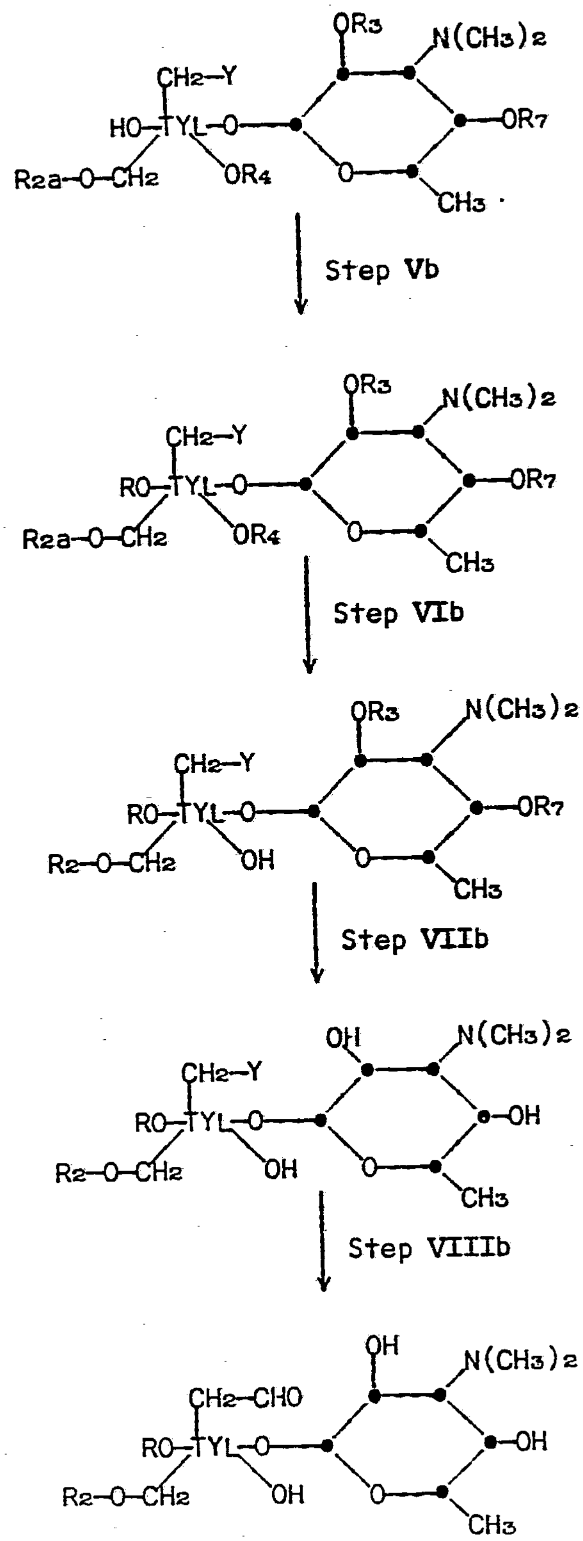


VIb

VIIb

VIIIb

IXb

Ib

where Y stands for a di(alkoxy)methyl group, a di(phenoxy)methyl group, a di(alkylthio)methyl group or a di(phenylthio)methyl group;

$R_3$ stands for a $C_{2-7}$ alkanoyl group;

$R_{2a}$ stands for a silylated mycinosyl group;

$R_4$ stands for a silyl group;

$R_5$ stands for a silyl group;

$R_6$ stands for hydrogen atom or a silyl group;

and

$R_7$ stands for a $C_{2-7}$ alkanoyl group.

The "alkyl" group in the di(alkoxy)methyl group and di(alkylthio)methyl group referred to by the symbol 'Y' is a $C_{1-4}$ residue of a straight chain or branched aliphatic hydrocarbon. The "phenyl" group in the di(phenoxy)methyl group and di(phenylthio)methyl group referred to by the symbol 'Y' is a phenyl group optionally substituted by a halogen atom such as chlorine atom, a $C_{1-4}$ alkyl group such as methyl or ethyl or a $C_{1-4}$ alkoxy group such as methoxy or ethoxy. The "$C_{2-7}$ alkanoyl" group referred to by the symbols '$R_3$' and '$R_7$' is the residue of an aliphatic carboxylic acid to be used as an acylating agent in the IIa or IIb. The "silylated mycinosyl" group of the symbol '$R_{2a}$' is the mycinosyl group in which the hydroxyl group on the 4'''-position is silylated by a silylating agent to be used in the step IIIa or IIIb. The "silyl" group referred to by the symbols '$R_4$', '$R_5$' and '$R_6$' is the silyl residue of a silylating agent to be used in the step IIIa or IIIb.

The process for preparing the tylosin derivatives of the general formula (I) (where $R_1$ is mycarosyl group) will be first described according to the Reaction Scheme A illustrated above.

Tylosin of formula (IIa) may be acetalized with an alcohol or thioacetalized with a thiol or a disulfide to protect the aldehyde group on the 20-position of the tylosin nucleus from the reactions which follow.

The alcohol to be employed in the step Ia as an acetalizing agent may include, for instance, an aliphatic alcohol or a phenol. The aliphatic alcohol may be, for instance, methyl alcohol, ethyl alcohol, propyl alcohol or the like, and the phenol may include, for instance, phenol, a halogenophenol such as chlorophenol, bromophenol or the like, a lower alkyl-substituted phenol such as cresol, xylenol, ethylphenol or the like, or a lower alkoxy-substituted phenol such as methoxyphenol, ethoxyphenol or the like. The thiols to be used in the step Ia as one of the thioacetalizing agents may include, for instance, a mercaptan or a thiophenol. Representatives of the mercaptans are, for instance, a lower alkyl mercaptan such as methylmercaptan, propylmercaptan or the like, and representatives of the thiophenols are, for instance, thiophenol, a halothiophenol such as chlorophenylmercaptan, bromophenylmercaptan or the like, a lower alkyl-substituted phenylmercaptan such as tolylmercaptan, xylylmercaptan, ethylphenylmercaptan, propylphenylmercaptan, isopropylphenylmercaptan or the like, or a lower alkoxy substituted phenylmercaptan such as methoxyphenylmercaptan,

ethoxyphenylmercaptan, propoxyphenylmercaptan or the like. The disulfides employed in step Ia as another thioacetalizing agent include, for instance, an alkyl disulfide, an aryl disulfide or the like. Representatives of the alkyl disulfides are, for instance, methyl disulfide, ethyl disulfide, propyl disulfide or the like, and representatives of the aryl disulfides are, for instance, phenyl disulfide, a substituted phenyl disulfide such as methylphenyl disulfide, methoxyphenyl disulfide or the like.

The acetalization or the thioacetalization according to the step Ia may be carried out in the presence of a solvent. As the solvents there may be employed, for instance, a chlorinated hydrocarbon such as chloroform or dichloromethane, acetonitrile, benzene, an ether or tetrahydrofuran. The acetalizing and thioacetalizing agents also may be employed as solvent. The reactions may be carried out at temperatures ranging from 0°C to the reflux temperature of the solvent used, for a period of time ranging from 15 minutes to 24 hours.

The thioacetalization according to the step Ia may be conducted in the presence of a tertiary phosphine such as a trialkylphosphine, for example, a trimethylphosphine, triethylphosphine or tributylphosphine or a triphenylphosphine such as triphenylphosphine.

The acetalization according to the step Ia provides the tylosin acetal of the general formula (IIIa) in which Y is a di(alkoxy)methyl group or a di(phenoxy)methyl group, and the thioacetalization according thereto provides the tylosin thioacetal of formula (IIIa) in which Y is a di(alkylthio)methyl group or a di(phenylthio)methyl group.

In step IIa, the tylosin acetal or thioacetal derivatives according to formula (IIIa) may be acylated with an acylating agent to thereby acylate the hydroxyl group on the 2'-position of the 5-mycaminosyl substituent. The acylating agent may be an acid anhydride or an acyl halide. The acid anhydride may include, for example, an anhydride of an aliphatic carboxylic acid such as acetic acid, propionic acid, butyric acid, valeric acid, caproic acid, isobutyric acid, isovaleric acid, and the acyl halide may include, for instance, a halide such as a chloride of the aliphatic carboxylic acids mentioned above.

The selective acylation of the 2'-hydroxy group is carried by conventional means in the absence of a base. As a solvent there may be employed a chlorinated hydrocarbon such as chloroform or dichloromethane. The reaction temperatures may vary from 0°C to 50°C, for example, room temperature, and the reaction time may range from 1 to 24 hours.

The acylation according to the step IIa provides the 2'-O-acylated tylosin acetal of formula (IVa) in which Y is a di(alkoxy)methyl group or a di(phenyloxy)methyl group and $R_3$ is a $C_{2-7}$ alkanoyl group or the 2'-O-acylated tylosin thioacetal of formula (IVa) in which Y is a di(alkylthio)methyl group or a di(phenylthio)methyl group and $R_3$ is a $C_{2-7}$ alkanoyl group.

The 2'-O-acylated tylosin acetal or thioacetal of formula (IVa) may then be silylated with a silylating agent in accordance with step IIIa. This silylation

will protect the hydroxyl groups which will interfere with or be acylated during the introduction of the acyl group into the 9-position of the tylosin nucleus.

The silylating agents to be used in the step IIIa may include, for instance, a tri-substituted halogenosilane or a hexa-substituted silazane. As the trisubstituted halogenosilane there may be employed a tri-lower alkyl halogenosilane such as trimethyl chlorosilane, triethyl chlorosilane or tributyl chlorosilane. As representatives of the hexasubstituted silazane, there may be employed a hexalower alkyl silazane such as hexamethyl silazane or hexaethyl silazane. As another type of silylating agent, there may be mentioned N-trimethyl silylimidazole, N,O-bis(trimethylsilyl)-acetamide, N,O-bis(trimethylsilyl)sulfamate and bis-trimethylsilyl urea. It is to be noted that the use of the trisubstituted halogenosilane or the hexasubstituted silazane as silylating agent does not silylate the hydroxyl group on the 3''-position of the mycarosyl substituent. Although the 3''-hydroxyl group of the mycarosyl substituent is not required to be silylated, because the 3''-hydroxyl group (that is a tertiary alcohol residue) is not acylated in the course of the acylation of the reduced hydroxy group on the 9-position of the tylosin nucleus, it is preferred to use N-trimethyl silylimidazole in a system where piperidine is present. This type of the silylating agent can permit silylation of the hydroxyl groups reactive in the acylation which follows, in addition to the 4''-hydroxyl group of the mycarosyl group.

In instances where the trisubstituted halosilane is employed, it is preferred to use a tertiary organic amine as an acid scavenger. The tertiary organic amine may include, for instance, pyridine, quinoline, N-methylmorpholine or dimethylaniline.

The silylation according to the step IIIa may be carried out in conventional manner, usually at a temperature below room temperature, for a period of time ranging preferably from approximately 20 to 40 minutes.

The silylation will give the silylated 2'-O-acyltylosin acetal or thioacetal of formula (Va) in which $R_4$ is a silyl group, $R_5$ is a silyl group and $R_6$ is hydrogen atom or a silyl group.

In the step IVa, the silylated 2'-O-acyl-tylosin acetal or thioacetal of formula (Va) is then reduced to convert the ketone group in the 9-position of the tylosin nucleus into a hydroxyl group.

The reducing agent to be used is one that can react exclusively with the ketone group in the 9-position, without reduction of the 1-lactone group double bonds in the macrolide ring. Such reducing agents include, for example, sodium borohydride and lithium alkoxyaluminum hydride.

The reduction according to step IVa may be conducted in conventional manner, usually in the presence of an inert solvent. The solvent to be used may be any aprotic solvent, for instance, an ether such as diethylene glycol, dimethylether or tetrahydrofuran. The reaction temperature is preferably below room temperature, and the reaction period is conventionally about 1 hour.

The 9-hydroxyl-2'-O-acyl-silyltylosin acetal or thioacetal of formula (VIa) produced in the step IVa is then subjected to acylation with an acylating agent according to the step Va. This acylation allows the alkanoyl residue of the acylating agent to be introduced into the 9-hydroxyl group of the tylosin nucleus.

The acylating agent to be used for the acylation according to the step Va may be an acid anhydride or an acyl halide. The acid anhydride may include, for instance, an anhydride of an aliphatic carboxylic acid such as acetic acid, propionic acid, butyric acid, valeric acid, caproic acid, isobutyric acid, isovaleric acid or the like or a benzoic acid which may optionally be substituted by a $C_{1-4}$ alkyl group such as methyl, ethyl or propyl, a halogen atom such as chlorine, a $C_{1-4}$ alkoxy group such as methoxy or ethoxy, or a nitro group, and the acyl halide may include, for instance, an acyl halide such as chloride of such an aliphatic acid or a benzoic acid as enumerated hereinabove.

The acylation according to the step Va may be carried out in conventional manner, usually in the presence of a base in a solvent. The base may include, for instance, a tertiary organic amine such as tribenzylamine, dimethylaniline, pyridine, picoline, collidine, N-methylpiperidine, N-methylmorpholine, quinoline or isoquinoline. The solvent to be used may include, for instance, a chlorinated hydrocarbon such as chloroform or methylene chloride, an aromatic hydrocarbon such as benzene or toluene, an ester such as ethyl acetate or butyl acetate, an ether such as tetrahydrofuran or dioxane, a ketone such as methylethylketone or methylisobutylketone. The tertiary amine to be used as a base also may be employed as a solvent.

The acylation according to the step Va is preferably below room temperature although temperatures from 0 to 100°C can be utilised. The reaction time may range from 1 to 24 hours. The acylation and reaction can be followed by thin layer chromatography.

In the step Va, the hydroxyl group in the 9-position of the 2'-O-acyl-silylation acetal or thioacetal of formula (VIa) is acylated to give the 9-O-acylated 2'-O-acyl-silyltylosin acetal or thioacetal of formula (VIIa), which in turn is subjected to elimination of the silyl groups in accordance with the step VIa.

The desilylation if preferably carried out in the presence of a $F^-$ donating reagent such as (n-butyl)$_4$NF or CsF. This reaction may also be conducted in a lower alkanol such as methanol or ethanol at elevated temperatures or in the presence of a base at room temperature. In instances where the desilylation is carried out at elevated temperatures, temperatures up to the reflux temperature of the solvent may be used. The base to be employed may be an alkali carbonate, triethylamine or the like. The use of the lower alkanol may also eliminate the alkanoyl group in the 2'-position of the 5-mycaminosyl substituent of the tylosin nucleus as well as the silyl groups. In order to desilylate the silyl groups from the 9-O-acylated 2'-acyl-silyl-tylosin acetal or thioacetal of formula (VIIa) with certainty, the use of the $F^-$ donating reagent is preferred.

The de-silylation according to the step VIa gives the 9-O-acylated 2'-O-acyltylosin acetal or thioacetal of formula (VIIIa) in which all the silyl groups are removed.

In step VIIa, the 9-O-acylated 2'-O-acyltylosin acetal or thioacetal of formula (VIIIa) may be deacylated to eliminate the acyl group on the 2'-position of the 5-mycaminosyl substituent. The deacylation may be carried out by refluxing in a lower alkanol such as methanol or ethanol.

The deacylation step VIIa provides the 9-O-acylated tylosin acetal or thioacetal of formula (IXa). The resulting compounds are then subjected to deacetalization or dethioacetalization according to step VIIIa.

The deacetalization or dethioacetalization of the 9-O-acylated tylosin acetal or thioacetal of formula (IXa) may be carried out by hydrolysis using an inert solvent such as tetrahydrofuran or acetonitrile at temperatures ranging from approximately 0°C to 60°C for a period of time ranging from approximately 30 minutes to 24 hours.

In instances where the hydrolysis is carried out with an acid catalyst such as a mineral acid, e.g., hydrochloric acid, the mycarosyl group on the 4'-position of the 5-mycaminosyl substituent will be eliminated simultaneously with the acetal or thioacetal group on the 20-position. It is thus to be noted that, in instances where the 9-O-acylated tylosin derivative according to formula (Ia) is desired to be obtained from the corresponding acetal or thioacetal of formula (IXa), mild reaction conditions should be selected so

as not to cause elimination of the mycarosyl group from the tylosin nucleus.

It is further to be noted that, in instances where the 9-O-acylated 2'-O-acyl-silyltylosin acetal of formula (VIIa) is treated under reaction conditions capable of selectively eliminating the 2'-acyl group, the silyl groups may also be eliminated and furthermore that, in instances where the acetal of formula (VIIa) is treated under reaction conditions capable of eliminating the acetal group, both the 2'-acyl and the silyl groups may be eliminated simultaneously with the acetal group. In these instances, however, it is to be understood that the mycarosyl group may also be eliminated from the 5-mycaminosyl substituent of the nucleus, whereby the corresponding demycarosyltylosin derivative is provided. In order to have all the protective groups eliminated with certainty, it is accordingly preferred to carry out the steps for eliminating the protective groups according to the reaction scheme as illustrated above.

The process for preparing the demycarosyltylosin derivatives of general formula (I) (where $R_1$ is hydrogen atom) will be described according to the Reaction Scheme B illustrated hereinabove.

In step Ib, the starting material to be used is demycarosyltylosin of formula (IIb) which may be produced by hydrolyzing tylosin of formula (IIa) with a mineral acid such as hydrochloric acid.

Demycarosyltylosin of formula (IIb) is acetalized or thioacetalized to have the acetal or thioacetal group introduced into the 20-aldehyde group

to give the demycarosyltylosin acetal or thioacetal of formula (IIIb).

The acetalization or thioacetalization of demycarosyltylosin according to the step Ib may be carried out in substantially the same manner as in step Ia in which tylosin is acetalized or thioacetalized.

In instances where the acetalization or thioacetalization is conducted with tylosin of formula (IIa) in the presence of a dilute acid such as acetic acid, the mycarosyl group may be eliminated from the tylosin to give the demycarosyltylosin acetal or thioacetal of the general formula (IIIb).

The demycarosyltylosin acetal or thioacetal of formula (IIIb) is then subjected to selective acylation on the 2'-hydroxyl group of the tylosin nucleus according to the step IIb. This selective acylation will also cause the acyl group to be introduced into the hydroxyl group in the 4'-position of the mycaminosyl substituent of the demycarosyltylosin compound as illustrated by the general formula (IVb). This reaction may be carried out in substantially the same manner as in the step IIa.

The 2',4'-O-acyldemycarosyltylosin acetal or thioacetal of formula (IVb) is then subjected to silylation in accordance with the step IIIb. The silylation may be carried out in substantially the same manner as in the step IIIa, thus introducing the silyl group into the hydroxyl groups on the 3- and 4''-positions of the tylosin nucleus to give the 2',4'-O-acyl-silyldemycarosyltylosin acetal or thioacetal of the general formula (Vb).

The following step IVb involves the reduction of the ketone group on the 9-position of the tylosin nucleus. The reduction may be conducted in substantially the same manner as in step IVa to give the 9-hydroxy-2',4'-O-acyl-silyldemycarosyltylosin acetal or thioacetal of the general formula (VIb).

In accordance with the step Vb which follows, the 9-hydroxy-2',4'-O-acylsilyldemycarosyltylosin acetal or thioacetal of formula (VIb) is then subjected to selective acylation in substantially the same manner as in step VIa. This reaction acylates the 9-hydroxyl group of the tylosin nucleus to provide the 9-O-acylated 2',4'-O-acyl-silyldemycarosyltylosin acetal or thioacetal of the general formula (VIIb).

In the steps which follow, the 9-O-acylated 2',4'-O-acyl-silyldemycarosyltylosin acetal or thioacetal of formula (VIIb) is subjected to a series of reactions which allow the hydroxy protecting groups to be eliminated. A series of reactions may be carried out as illustrated in Reaction Scheme B above.

Step VIb involves the desilylation which may be carried out in substantially the same manner as in step VIa described above. This reaction may eliminate the 3-O-silyl group ($OR_4$) and 4'''-O-silyl group of the mycinosyl group ($R_{2a}$) from the 9-O-acylated 2',4'-O-acylsilyldemycarosyltylosin acetal or thioacetal of formula (VIIb).

The 9-O-acylated 2',4'-O-acyldemycarosyltylosin acetal or thioacetal of formula (VIIIb) obtained by the step VIb may then be subjected to selective deacylation of the acyl groups from the 2'- and 4'-alkanoyloxy

groups of the mycaminosyl substituent on the 5-position of the tylosin nucleus with the 9-O-acyl group retained without removal. The deacylation may be carried out in substantially the same manner as in the step VIIa described hereinabove.

The 9-O-acylated demycarosyltylosin acetal or thioacetal of formula (IXb) thus obtained is then subjected to deacetalization or dethioacetalization according to step VIIIb. This reaction may be carried out in substantially the same manner as in step VIIIa described hereinabove, thereby eliminating the acetal or thioacetal group (V) to provide the 9-O-acylated demycarosyltylosin derivative of general formula (Ib).

It is further to be noted that in those instances where the acetal group is eliminated from the corresponding acetal derivatives, the reaction conditions are substantially the same as with the reaction in which the silyl groups are removed. Accordingly, in Reaction Scheme B in which acetal derivatives are employed, steps VIb and VIIIb may be combined and carried out simultaneously as one step. In this instance, the acyl groups on the 2'- and 4'-positions are deacylated selectively in accordance with the step VIIb.

In instances where the thioacetal derivatives are employed, the thioacetal group may not be removed under those reaction conditions where the silyl groups are eliminated from the silylated demycarosyltylosin thioacetal derivatives. It is thus necessary to remove the thioacetal group selectively with the aid of a dethioacetalizing agent such as HgO.

Thus, the compounds of formula (I) can be prepared by acylating a 9-hydroxytylosin derivative in which the 20-aldehyde and free hydroxyl groups susceptible to acylation have been protected, followed by removal of the groups used to protect said aldehyde and hydroxyl groups.

The tylosin derivatives of formula (I) may be used as a pharmaceutically-acceptable salt such as an acid addition salt with an organic acid, e.g., tartaric acid, acetic acid, propionic acid, lactic acid, citric acid or succinic acid or an inorganic acid, e.g. hydrochloric acid, sulfuric acid or phosphoric acid. The pharmaceutically-acceptable salts may be prepared in conventional manner.

The tylosin derivatives represented by the general formula (I), and pharmaceutically-acceptable salts thereof are particularly effective against gram-positive bacteria including some strains showing resistance to macrolide antibiotics. Accordingly, the tylosin derivatives according to the present invention can be used as human pharmaceuticals or veterinary drugs for prevention or treatment of bacterial infections.

The antibacterial composition according to the present invention capable of being employed for prevention and treatment of bacterial infections as described hereinabove contains the tylosin derivative represented by the general formula (I) as an active ingredient. The antibacterial composition according to the present invention may be administered as solid preparations such as capsules, tablets, granules or the like or as liquid preparations such as solutions,

suspensions or the like through oral routes, rectal or parenteral routes. The preparations as described hereinabove may contain a conventional additive such as an excipient, binder, suspending agent, emulsifying agent, dissolution acid or buffer.

The dosage may range from 1 mg/kg to 100 mg/kg per single dose when administered in humans, and from 1 mg/kg to 1,000 mg/kg per single dose when administered as a veterinary drug.

The present invention will be described more in detail by way or working examples.

<u>Preparation Example 1</u>: Tylosin dimethylacetal

To a solution of 1 gram of tylosin in 20 ml of methanol was added 0.34 ml of difluoroacetic acid, and the mixture was allowed to stand at room temperature for 2 days. After the reaction was over, the reaction mixture was neutralized by sodium hydrogen carbonate, and methanol was then distilled off under reduced pressure. To the residue was added 50 ml of water, and the resulting solution was extracted with three 50 ml portions of ethyl acetate. The extract was dried over anhydrous sodium sulfate and concentrated under reduced pressure to leave 800 mg of a pale yellow crude powder which in turn was purified by means of silica gel column chromatography using a $CHCl_3$:$CH_3OH$ (10:1) mixture. Tylosin dimethylacetal was obtained with the yield of about 400 mg.

Mass Spectrum: (m/z) 961 ($M^+$)

Proton NMR Spectrum:

$\delta$(ppm) 3.25, 3.30 (each 3Hs, C-20$(OCH_3)_2$)

Preparation Example 2: Tylosin diphenylthioacetal

To a solution of 10 grams of tylosin and 3.6 grams of phenyl disulfide in 80 ml of chloroform was added 4.5 ml of tri-n-butylphosphine under a nitrogen atmosphere, and the mixture was allowed to react at room temperature for 2 hours. After the reaction was over, the resulting reaction mixture was concentrated under reduced pressure, and the residue was purified by means of silica gel chromatography using a chloroform:methanol:concentrated ammonia (50:1:0.05) mixture to yield 7.9 grams (65%) of tylosin diphenylthioacetal.

Mass Spectrum: (m/z) 1117 ($M^+$), 608 (aglycone), 318 (mycarosylmycaminose), 191 (mycinose)

Proton NMR Spectrum:

δ(ppm) 1.77 (3Hs, $N(CH_3)_2$)

2.57 (6Hs, $N(CH_3)_2$)

3.45, 3.59 (each 3Hs, 2'''-$OCH_3$, 3'''-$OCH_3$)

4.16 (1Hd, J=7.5 Hz, $H_1$')

4.54 (1Hd, J=8.0 Hz, $H_1$''')

5.0 (b, H15)

5.06 (b, $H_1$'')

5.83 (1Hd, J=9.5 Hz, $H_{13}$)

6.23 (1Hd, J=13.5 Hz, $H_{10}$)

7.1~7.6 (m, $H_{11}$, aromatic ring proton)

Preparation Example 3: Demycarosyltylosin dimethylacetal

A solution of 10 grams of tylosin in 100 ml of a 0.5% hydrogen chloride-methanol mixture was allowed to react at room temperature for 1 hour. After the reaction was over, the resulting reaction mixture was poured into a saturated sodium hydrogen carbonate aqueous solution, and the mixture was extracted with chloroform. The resulting chloroform layer was dried over anhydrous sodium sulfate and concentrated to dryness under reduced pressure to give a crude powder. It was then subjected to silica gel column chromatography using a chloroform:methanol:concentrated ammonia (50:1:0.05) mixture to yield 8.5 grams (95%) of demycarosyl-tylosin dimethylacetal.

Mass Spectrum: (m/z) 817 ($M^+$), 468 (aglycone), 190,174 (mycaminose)

Proton NMR Spectrum:

δ(ppm) 1.69 (3Hs, $H_{22}$)
2.49 (6Hs, $N(CH_3)_2$)
3.23, 3.28 (each 3Hs, acetl-$CH_3$)
3.45, 3.58 (each 3Hs, 2''-$OCH_3$, 3''-$OCH_3$)
4.30 (1Hd, J=7.5 Hz, $H_1$')
4.55 (1Hd, J=7.5 Hz, $H_1$'')
4.96 (1Hbt, $J_{15,16}$=7.5 Hz, $H_{15}$)
5.86 (1Hbd, J=11.0 Hz, $H_{13}$)
6.25 (1Hd, J=15.0 Hz, $H_{10}$)
7.28 (1Hd, J=15.0 Hz, $H_{11}$)

Preparation Example 4: Demycarosyltylosin diphenylthioacetal

(a) The procedures of Example 2 were followed with the exception that demycarosyltylosin was used instead of tylosin to give demycarosyltylosin diphenylthioacetal.

(b) The procedures of Example 2 were followed with the exception that the reaction was carried out with the aid of acetic acid to give demycarosyltylosin diphenylthioacetal.

Example 1: 9-O-Acetyl-9-dihydrotylosin

To a solution of 10 grams of tylosin diphenylthioacetal in 100 ml of chloroform was added 4.6 ml of acetic anhydride. The reaction mixture was poured into a cold saturated sodium hydrogen carbonate aqueous solution and then extracted with chloroform. The extract was dried over anhydrous sodium sulfate and concentrated to dryness under reduced pressure to give 9.9 grams of 2'-O-acetyltylosin diphenylthioacetal as a colorless solid.

The solid product obtained was dissolved in 7.4 ml of piperidine, and 7.4 ml of N-trimethylsilylimidazole was added to the solution. The mixture was allowed to stand at room temperature for 30 minutes, and the reaction mixture was diluted with chloroform and washed with a saturated sodium chloride solution. The resulting chloroform layer was dried over anhydrous sodium sulfate and concentrated to dryness under reduced

pressure to leave a crude powder which in turn was purified by means of silica gel column chromatography using a benzene:acetone (35:1) mixture. The yield of 2'-O-acetyl-3,3'',4'',4'''-tetra-O-trimethylsilyltylosin diphenylthioacetal was 4.1 grams, while 2'-O-acetyltylosin diphenylthioacetal was obtained as a pure product in the amount of 6.0 grams.

Mass Spectrum: (m/z) 1447 ($M^+$)

Proton NMR Spectrum: δ(ppm) 0.1-0.2 (s, $Si(CH_3)_3$)

1.76 (3Hs, H-22)

2.03 (3Hs, 2'-$OCOCH_3$)

2.38 (6Hs, 3'-$N(CH_3)_2$)

3.46 (3Hs, 2'''-$OCH_3$)

3.56 (3Hs, 3'''-$OCH_3$)

4.56 (1Hd, J=8.0 Hz, H-1''')

4.84 (1Hd, J=12.0 Hz, H-13)

6.20 (1Hd, J=15.0 Hz, H-10)

To a solution of 2.4 grams of 2'-O-acetyl-3,3'',4'',4'''-tetra-O-trimethylsilyltylosin diphenylthioacetal in 20 ml of diethyleneglycol dimethylether was added 83 mg of sodium borohydride, and the mixture was stirred at room temperature for 20 hours. The resulting reaction mixture was diluted with chloroform and washed with a saturated sodium chloride aqueous solution. The resulting chloroform layer was dried over anhydrous sodium sulfate and concentrated to dryness under reduced pressure to give a crude material which in turn was purified by means of silica gel column chromatography using a benzene:acetone (30:1)

mixture, thus leaving 1.1 grams of 2'-O-acetyl-3,3'',4'',4'''-tetra-O-trimethylsilyl-9-dihydrotylosin diphenylthioacetal.

Mass Spectrum: (m/z) 1449 ($M^+$)

Proton NMR Spectrum: δ(ppm) 0.1 (s, $-OSi(CH_3)_3$)

1.66 (3Hs, H-22)

2.00 (3Hs, 2'-$OCOCH_3$)

2.40 (6Hs, 3'-$N(CH_3)_2$)

3.46 (3Hs, 2'''-$OCH_3$)

3.56 (3Hs, 3'''-$OCH_3$)

5.46 (1Hd, J=10.0 Hz, H-13)

5.58 (1Hdd, $J_{9,10}$=4.0 Hz, $J_{10,11}$=15.0 Hz, H-10)

6.10 (1Hd, J=15.0 Hz, H-11)

To a solution of 150 mg of 2'-O-acetyl-3,3'',4'',4'''-tetra-O-trimethylsilyl-9-dihydrotylosin diphenylacetal in 2 ml of pyridine was added 0.6 ml of acetic anhydride, and the mixture was allowed to stand at room temperature for 6 hours. The reaction mixture was then poured into a cold saturated sodium hydrogen carbonate aqueous solution, and the mixture was extracted with chloroform. The resulting chloroform layer was dried over anhydrous sodium sulfate and concentrated to dryness under reduced pressure. The resulting crude material was dissolved in a solution of 0.5 ml of a 1N tetrahydrofuran solution of tetrabutylammonium fluoride, and the solution was allowed to stand at room temperature for 30 minutes. The reaction mixture was diluted with chloroform, washed with a saturated

sodium hydrogen carbonate aqueous solution, dried over anhydrous sodium sulfate, and concentrated to dryness under reduced pressure to give 9,2'-di-O-acetyl-9-dihydrotylosin diphenylthioacetal.

The product was then dissolved in 5 ml of methanol and refluxed for 66 hours. After the methanol was distilled off under reduced pressure, the residue was diluted in chloroform, washed with a saturated sodium hydrogen carbonate aqueous solution, dried over anhydrous sodium sulfate, and concentrated to dryness under reduced pressure to give 9-O-acetyl-9-dihydrotylosin diphenylthioacetal as a crude material.

The crude material was then dissolved in 5 ml of a 15% aqueous tetrahydrofuran solution, and 50 mg of mercuric oxide (red) and 35 µl of boron trifluoride ether complex were added. The mixture was stirred at room temperature for 1 hour, and the resulting reaction mixture was poured into a saturated sodium hydrogen carbonate aqueous solution. After extraction with diethyl ether, the extract was dried over anhydrous sodium sulfate and concentrated to dryness under reduced pressure to leave a crude powder. The crude material was then purified by means of silica gel thin layer chromatography with chloroform:methanol:concentrated ammonia (15:1:0.05) mixture to give 29 mg of 9-O-acetyl-9-dihydrotylosin.

$[\alpha]_D^{28}$ -64.5° (c 1.0, $CH_3OH$)

UV: $\lambda_{max}^{CH_3OH}$ nm(ε) 235 (18,500)

Mass Spectrum: (m/z) 815 ($M^+$ -mycarose) 625 ($M^+$ -mycarosylmycaminose)

Proton NMR Spectrum: δ(ppm) 1.70 (3Hs, H-22)

2.06 (3Hs, $COCH_3$)

2.47 (6Hs, $N(CH_3)_2$)

3.43 (3Hs, 2'''-$OCH_3$)

3.57 (3Hs, 3'''-$OCH_3$)

4.27 (1Hd, J=8.0 Hz, H-1')

4.51 (1Hd, J=8.0 Hz, H-1''')

4.95 (1Hbt, H-15)

5.05 (1Hd, J=3.0 Hz, H-1'')

5.36 (1Hd, J=10.0 Hz, H-13)

5.74 (1Hdd, $J_{9,10}$=3.0 Hz, $J_{10,11}$=16.5 Hz, H-10)

6.20 (1Hd, J=16.5 Hz, H-11)

9.70 (1Hs, H-20)

<u>Example 2</u>: 9-O-Propionyl-9-dihydrotylosin

The procedures of Example 1 were followed with the exception that 0.7 ml of propionic anhydride was used instead of acetic anhydride. The resulting product was then treated to eliminate the protective groups in substantially the same manner as in Example 1 to give 30 mg of 9-O-propionyl-9-dihydrotylosin.

$[\alpha]_D^{23}$ -80.3° (c 1.0, $CH_3OH$)

UV: $\lambda_{max}^{CH_3OH}$ nm(ε) 235 (25,700)

Mass Spectrum: (m/z) 829 ($M^+$ -mycarose)

Proton NMR Spectrum: δ(ppm) 1.69 (3Hs, H-22)

2.53 (6Hs, $N(CH_3)_2$)

3.43 (3Hs, 2'''-$OCH_3$)

3.57 (3Hs, 3'''-$OCH_3$)

4.27 (1Hd, J=7.5 Hz, H-1')

4.50 (1Hd, J=7.5 Hz, H-1''')

4.95 (1Hbt, $J_{15,16}$=7.5 Hz, H-15)

5.05 (1Hd, J=3.0 Hz, H-1'')

5.33 (1Hd, J=10.5 Hz, H-13)

5.76 (1Hdd, $J_{9,10}$=4.0 Hz, $J_{10,11}$=16.0 Hz, H-10)

6.18 (1Hd, J=16.0 Hz, H-11)

9.68 (1Hs, H-20)

Example 3: 9-O-Butyryl-9-dihydrotylosin

Using 0.9 ml of butyric anhydride instead of acetic anhydride, the procedures of Example 1 were followed to give 22 mg of 9-O-butyryl-9-dihydrotylosin.

$[\alpha]_D^{23}$ -72.5° (c 1.0, $CH_3OH$)

UV: $\lambda_{max}^{CH_3OH}$ nm(ε) 235 (20,600)

Mass Spectrum: (m/z) 969 ($M^+$ $-H_2O$), 842 ($M^+$ -mycarose), 812 ($M^+$ -mycinose)

Proton NMR Spectrum: δ(ppm) 1.69 (3Hs, H-22)

2.49 (6Hs, $N(CH_3)_2$)

3.43 (3Hs, 2'''-$OCH_3$)

3.57 (3Hs, 3'''-$OCH_3$)

4.27 (1Hd, J=7.5 Hz, H-1')

4.50 (1Hd, J=7.5 Hz, H-1''')

4.95 (1Hbt, H-15)

5.05 (1Hd, J=3.0 Hz, H-1'')

5.33 (1Hd, J=10.5 Hz, H-13)

5.76 (1Hd, $J_{9,10}$=4.5 Hz, $J_{10,11}$=15.0 Hz, H-10)

6.38 (1Hd, J=15.0 Hz, H-11)

9.68 (1Hs, H-20)

Example 4: 9-O-Benzoyl-9-dihydrotylosin

Using 0.5 ml of benzoyl chloride instead of acetic anhydride, the procedures of Example 1 were followed to give 57 mg of 9-O-benzoyl-9-dihydrotylosin.

$[\alpha]_D^{23}$ -46.8° (c 1.0, $CH_3OH$)

UV: $\lambda_{max}^{CH_3OH}$ nm(ε) 228 (24,100)

Mass Spectrum: (m/z) 687 ($M^+$ -mycarosyl-mycaminose)

Proton NMR Spectrum: δ(ppm) 1.72 (3Hs, H-22)

2.5 (6Hs, $N(CH_3)_2$)

3.38 (3Hs, 2'''-$OCH_3$)

3.54 (3Hs, 3'''-$OCH_3$)
4.35 (1Hd, J=8.0 Hz, H-1')
4.48 (1Hd, J=8.0 Hz, H-1''')
4.93 (1Hbt, H-15)
5.08 (1Hbs, H-1'')
5.35 (1Hb, J=10.0 Hz, H-13)
5.92 (1Hdd, $J_{9,10}$=4.6 Hz, $J_{10,11}$=16.0 Hz, H-10)
6.34 (1Hd, J=16.0 Hz, H-11)
7.5-8.1 (5Hm, aromatic proton)

Example 5: 9-O-Acetyl-9-dihydrodemycarosyltylosin

To a solution of 10 grams of demycarosyltylosin dimethylacetal in 100 ml of chloroform was added 4.6 ml of acetic anhydride, and the mixture was allowed to stand at room temperature for 2 hours. The reaction mixture was poured into a cold saturated sodium hydrogen aqueous solution and the mixture was extracted with chloroform. The extract was dried over anhydrous sodium sulfate and concentrated to dryness under reduced pressure to give 2',4'-di-O-acetyldemycarosyltylosin dimethylacetal as a crude material.

The crude material obtained hereinabove was dissolved in 7 ml of peperidine, and 7 ml of N-trimethylsilylimidazole were added to the solution. The mixture was allowed to stand at room temperature for 30 minutes, and the reaction mixture was diluted with chloroform, washed with a saturated sodium chloride solution, dried over anhydrous sodium sulfate, and concentrated to dryness under reduced pressure. The

resulting crude powder was purified by means of silica gel column chromatography using a benzene:acetone (20:1) mixture to give 5.3 grams of 2',4'-di-O-acetyl-3,4''-di-O-trimethylsilyldemycarosyltylosin dimethylacetal.

Mass Spectrum: (m/z) 1447 ($M^+$)

Proton NMR Spectrum: δ(ppm) 0.10, 0.17 (each 9Hs, $-OSi(CH_3)_3$)

1.76 (3Hs, H-22)

2.05 (6Hs, 2',4'-$OCOCH_3$)

2.34 (6Hs, 3'-$N(CH_3)_2$)

3.20, 3.29 (each 3Hs, 20-$(OCH_3)_2$)

3.47 (3Hs, 2''-$OCH_3$)

3.59 (3Hs, 3''-$OCH_3$)

4.38 (1Hd, J=7.6 Hz, H-1'')

4.58 (1Hd, J=7.8 Hz, H-1')

5.87 (1Hd, J=10.3 Hz, H-13)

6.24 (1Hd, J=15.2 Hz, H-10)

7.21 (1Hd, J=15.2 Hz, H-11)

To a solution of 1 gram of 2',4'-di-O-acetyl-3,4''-di-O-trimethylsilyldemycarosyltylosin dimethylacetal in 5 ml of diethyleneglycol dimethyl ether were added 30 ml of sodium borohydride, and the mixture was stirred at room temperature for 20 hours. The reaction mixture was diluted with chloroform, washed with a saturated sodium chloride solution, dried over anhydrous sodium sulfate, and concentrated to dryness under reduced pressure. The residue was then purified by means of silica gel column chromatography using a benzene:acetone (20:1) mixture to give 2',4'-di-O-acetyl-3,4''-di-O-trimethylsilyl-9-dihydrodemycarosyl-tylosin dimethylacetal.

Mass Spectrum: (m/z) 1047 ($M^+$)

Proton NMR Spectrum: δ(ppm) 0.16 (18 Hs, $-Si(CH_3)_3$)

1.70 (3Hs, H-22)

2.03, 2.05 (each 3Hs, 2',4'-$OCOCH_3$)

3.30, 3.44 (each 3Hs, 20-$(OCH_3)_2$)

3.47 (3Hs, 2''-$OCH_3$)

3.59 (3Hs, 3''-$OCH_3$)

4.47 (1Hd, J=8.6 Hz, H-1')

4.57 (1Hd, J=9.1 Hz, H-1'')

5.37 (1Hd, J=10.5 Hz, H-13)

5.68 (1Hdd, $J_{9,10}$=3.2 Hz, $J_{10,11}$=15.4 Hz)

6.34 (1Hd, J=15.4 Hz, H-11)

To a solution of 350 mg of 2',4'-di-O-acetyl-3,4''-di-O-trimethylsilyl-9-dihydrodemycarosyltylosin dimethylacetal in 2 ml of pyridine was added 1 ml of acetic anhydride, and the mixture was allowed to stand at room temperature for 6 hours. The reaction mixture was mixed with 5 ml of methanol and then refluxed for 2 hours. After concentration to dryness under reduced pressure, the residue was dissolved in 5 ml of acetonitrile and 1 ml of 1N HCl was added. The reaction mixture was stirred for 1 hour, poured into a cold saturated sodium hydrogen carbonate aqueous solution and extracted with chloroform. The extract was dried over anhydrous sodium sulfate and concentrated to dryness under reduced pressure to leave a crude powder which in turn was purified by means of silica gel

column chromatography using a chloroform:methanol (20:1) mixture to give 194 mg of 9-O-acetyl-9-dihydrodemycarosyltylosin.

$[\alpha]_D^{28}$ -46.8° (c 1.0, $CH_3OH$)

UV: $\lambda_{max}^{CH_3OH}$ nm(ε) 231 (27,800)

Mass Spectrum: (m/z) 797 ($M^+$ -18)

Proton NMR Spectrum: δ(ppm) 1.71 (3Hs, H-22)

2.08 (3Hs, 9-$OCOCH_3$)

2.50 (6Hs, 3'-$N(CH_3)_2$)

3.47 (3Hs, 2''-$OCH_3$)

3.61 (3Hs, 3''-$OCH_3$)

4.34 (1Hd, J=7.5 Hz, H-1')

4.54 (1Hd, J=8.0 Hz, H-1'')

5.40 (1Hd, J=10.5 Hz, H-13)

5.78 (1Hdd, $J_{9,10}$=4.0 Hz, $J_{10,11}$=16.0 Hz, H-10)

6.24 (1Hd, J=16.0 Hz, H-11)

9.74 (1Hs, H-20)

Example 6: 9-O-Propionyl-9-dihydrodemycarosyltylosin

To a solution of 350 mg of 2,4'-di-O-acetyl-3,4''-di-O-trimethylsilyl-9-dihydrodemycarosyltylosin dimethylacetal obtained in Example 5 in 2 ml of pyridine was added 1 ml of propionic anhydride, and the mixture was allowed to stand at room temperature for 6 hours to give 9-O-propionyl-2',4'-di-O-acetyl-3,4'-di-O-trimethylsilyl-9-dihydrodemycarosyltylosin dimethylacetal.

The resulting acetal was then treated in substantially the same manner as in Example 5 to give 185 mg of 9-O-propionyl-9-dihydrodemycarosyltylosin.

$[\alpha]^{28}_{D}$ -53.4° (c 1., $CH_3OH$)

UV: $\lambda^{CH_3OH}_{max}$ nm(ε) 240 (30,500)

Mass Spectrum: m/z 639 ($M^+$ -mycaminose)

Proton NMR Spectrum: δ(ppm) 1.70 (3Hs, H-22)

2.49 (6Hs, 3'-$N(CH_3)_2$)

3.46 (3Hs, 2''-$OCH_3$)

3.61 (3Hs, 3''-$OCH_3$)

4.34 (1Hd, J=7.5 Hz, H-1')

4.54 (1Hd, J=8.0 Hz, H-1'')

5.37 (1Hd, J=10.0 Hz, H-13)

5.79 (1Hdd, $J_{9,10}$=4.0 Hz, $J_{10,11}$=16.0 Hz, H-10)

6.22 (1Hd, J=16.0 Hz)

9.73 (1Hs, H-20)

Example 7: 9-O-Butyryl-9-dihydrodemycarosyltylosin

2',4'-Di-O-acetyl-3,4''-di-O-trimethylsilyl-9-dihydrodemycarosyltylosin dimethylacetal (350 mg) was dissolved in 2 ml of pyridine, and 1 ml of butyric anhydride was added to the solution. The resulting mixture was then allowed to stand at room temperature for 6 hours to give 9-O-butyryl-2',4'-di-O-acetyl-3,4''-di-O-trimethylsilyl-9-dihydrodemycarosyltylosin dimethylacetal which in turn was treated in substantially

the same manner in Example 5 to give 180 mg of 9-O-butyryl-9-dihydrodemycarosyltylosin.

$[\alpha]_D^{28}$ 52° (c 1, $CH_3OH$)

UV: $\lambda_{max}^{CH_3OH}$ nm(ε) 238 (30,000)

Mass Spectrum: (m/z) 653 ($M^+$ -mycaminose)

Proton NMR Spectrum: δ(ppm) 1.71 (3Hs, H-22)

2.50 (6Hs, 3'-$N(CH_3)_2$)

3.46 (3Hs, 2''-$OCH_3$)

3.60 (3Hs, 3''-$OCH_3$)

4.34 (1Hd, J=7.5 Hz, H-1')

4.54 (1Hd, J=8.0 Hz, H-1'')

5.37 (1Hd, J=10.5 Hz, H-13)

5.79 (1Hdd, $J_{9,10}$=4.0 Hz, $J_{10,11}$=16.0 Hz, H-10)

6.23 (1Hd, J=16.0 Hz, H-11)

9.73 (1Hs, H-20)

Example 8: 9-O-Benzoyl-9-dihydrodemycarosyltylosin

2',4'-Di-O-acetyl-O-trimethylsilyl-9-dihydrodemycarosyltylosin dimethylacetal obtained in Example 5 was dissolved in 1 ml of pyridine, and 0.3 ml of benzoyl chloride was added to the solution. The resulting mixture was allowed to stand at room temperature for 6 hours to give 9-O-benzoyl-2',4'-di-O-acetyl-3,4''-di-O-trimethylsilyl-9-dihydrodemycarosyltylosin dimethylacetal. This acetal was then treated in substantially the same manner as in Example 5 to give 190 mg of 9-O-benzoyl-9-benzoyl-9-dihydrodemycarosyltylosin.

$[\alpha]_D^{21}$ -38° (c 1., $CH_3OH$)

UV: $\lambda_{max}^{CH_3OH}$ nm(ε) 229 (23,100)

Mass Spectrum: (m/z) 756 ($M^+$ -O or $PhCO_2$)

Proton NMR Spectrum: δ(ppm) 1.73 (3Hs, H-22)

2.55 (6Hs, 3'-$N(CH_3)_2$)

3.33 (3Hs, 2''-$OCH_3$)

3.54 (3Hs, 3''-$OCH_3$)

4.38 (1Hd, J=8.8 Hz, H-1')

4.48 (1Hd, J=7.8 Hz, H-1'')

5.33 (1Hd, J=10.3 Hz, H-13)

5.92 (1Hdd, $J_{9,10}$=3.9 Hz, $J_{10,11}$=15.9 Hz, H-10)

6.33 (1Hd, J=15.9 Hz, H-11)

7.3-7.7 (3Hm, aromatic protons)

7.9-7.2 (2Hm, aromatic protons)

9.67 (1Hs, H-20)

Example 9:

Using the acetal or thioacetal obtained in each of Preparation Example 2 through 4, the procedures of each of Examples 1 through 8 were followed in substantially the same manner to give the corresponding products as given in each of Examples 1 through 8.

Experiment 1: Minimum Inhibitory Concentration (μg/ml)

The tylosin derivatives obtained in Examples 1 through 9 were tested for their minimum inhibitory concentrations against gram-positive and gram-negative bacteria. The tests were conducted in conventional manner by inoculating varying concentrations of the test sample on a nutrient agar test medium and incubating at 37°C for 18-20 hours. The concentrations of the test samples inhibiting the bacteria growth were measured in μg per ml as a minimum inhibitory concentration. The test results are shown below:

**Table**

**Minimum Inhibitory Concentrations (μg/ml)**

| Test Organisms | Ex.1 | Ex.2 | Ex.3 | Ex.4 | Ex.5 | Ex.6 | Ex.7 | Ex.8 | Tyl | Detyl |
|---|---|---|---|---|---|---|---|---|---|---|
| *Staphylococcus aureus* KB-210 (ATC 65380) | 1.56 | 0.78 | 1.56 | 0.78 | 1.56 | 1.56 | 1.56 | 1.56 | 1.56 | 3.12 |
| *Bacillus subtilis* KB-143(IFO 3001) | 0.2 | 0.2 | 0.4 | 0.2 | 1.56 | 1.56 | 1.56 | 1.56 | 0.78 | 3.12 |
| *Micrococcus luteus* KB212 (ATCC 9341) | <0.1 | <0.1 | <0.1 | <0.1 | 0.2 | 0.2 | 0.2 | 0.2 | 0.2 | 0.2 |
| *Escherichia coli* KB213 (NIHJ) | >100 | >100 | 100 | >100 | >50 | >100 | >50 | >100 | >100 | >100 |
| *Klebsiella pneumoniae* KB13(PCI 602) | 100 | >100 | 100 | >100 | >50 | >100 | >50 | >100 | >100 | 100 |

Notes: Tyl = Tylsoin
Detyl = Demycarosyltylosin

Experiment 2: *In vivo* Activity

The compounds obtained in Examples 2 and 4 were separately administered subcutaneously or orally to mice infected with *S. pyrogenes* C203. The *in vivo* activity was measured as $ED_{50}$ (mg/kg x 2). The result are shown below:

Table

| Compounds | Subcutaneous | Oral |
|---|---|---|
| Tylosin | 1.0 | 50 |
| Example 2 | 2.5 | 12.5 |

## CLAIMS

1. A compound of formula (I):

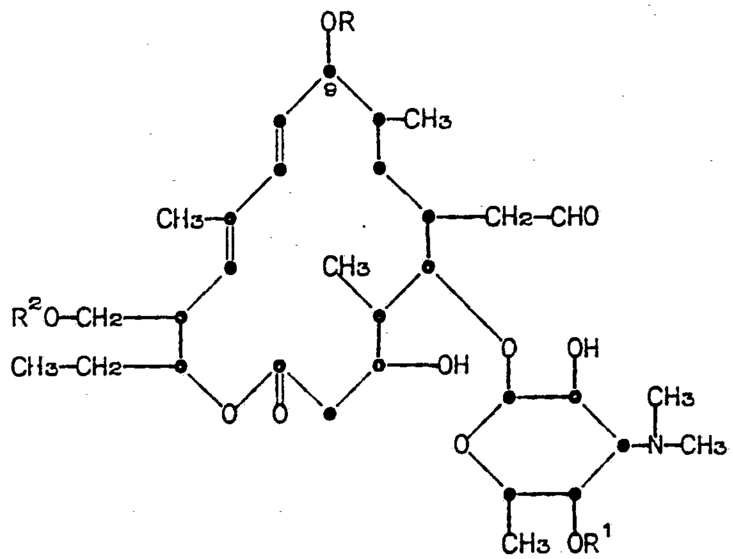

(I)

where R is a $C_{2-7}$ alkanoyl or benzoyl group optionally substituted by $C_{1-4}$ alkyl, $C_{1-4}$ alkoxy, nitro or halo;

where $R^1$ is hydrogen or mycarosyl; and

$R^2$ is mycinosyl;

or a pharmaceutically-acceptable salt thereof.

2. 9-O-Acetyl-9-dihydrotylosin.

3. 9-O-Propionyl-9-dihydrotylosin.

4. 9-O-Benzoyl-9-dihydrotylosin.

5. 9-O-Acetyl-9-dihydrodemycarosyltylosin.

6. 9-O-Propionyl-9-dihydrodemycarosyltylosin.

7. A compound of formula (I), or a pharmaceutically-acceptable salt thereof, as claimed in any

0194793



one of claims 1 to 6, for use in the prophylaxis or therapy of bacterial infections in warm blooded animals.

8. A pharmaceutical or veterinary formulation comprising as an active ingredient a compound of formula (I), or a pharmaceutically-acceptable salt thereof, as claimed in any one of claims 1 to 6, associated with one or more pharmaceutically or veterinarally acceptable carriers therefor.

9. A process for preparing a compound of formula (I), or a pharmaceutically-acceptable salt thereof, as claimed in any one of claims 1 to 6, which comprises acylating a 9-hydroxytylosin derivative of formula:

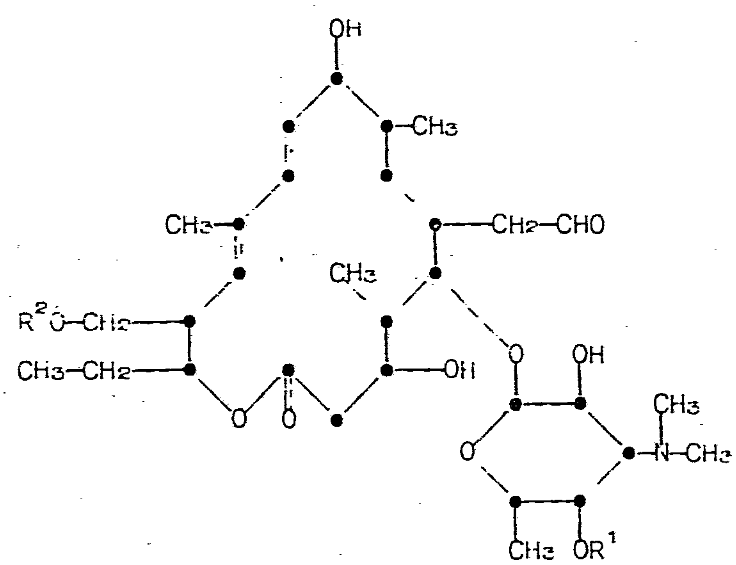

where $R^1$ and $R^2$ are as defined in claim 1, and in which the 20-aldehyde and free hydroxyl groups susceptible to acylation have been protected, followed by removal of the groups used to protect said aldehyde and hydroxyl groups.

## CLAIMS

1. A process for preparing a compound of formula (I):

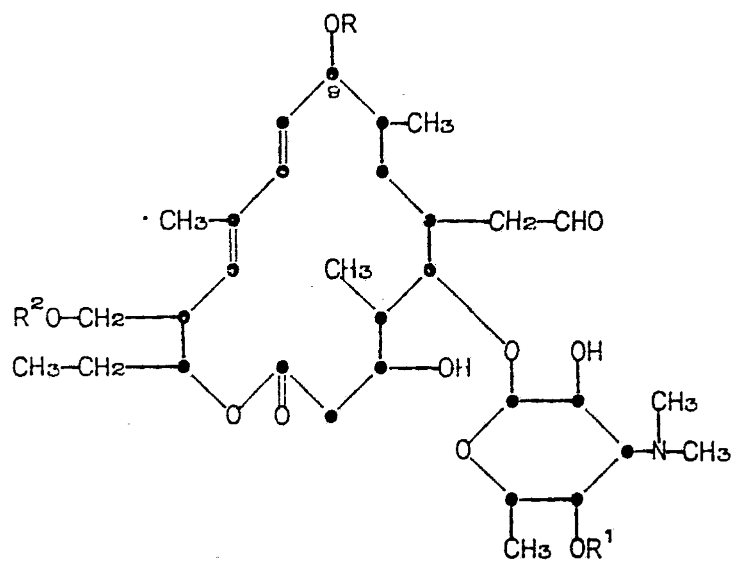

(I)

where R is a $C_{2-7}$ alkanoyl or benzoyl group optionally substituted by $C_{1-4}$ alkyl, $C_{1-4}$ alkoxy, nitro or halo;

where $R^1$ is hydrogen or mycarosyl; and

$R^2$ is mycinosyl;

or a pharmaceutically-acceptable salt thereof; which comprises acylating a 9-hydroxytylosin derivative of formula:

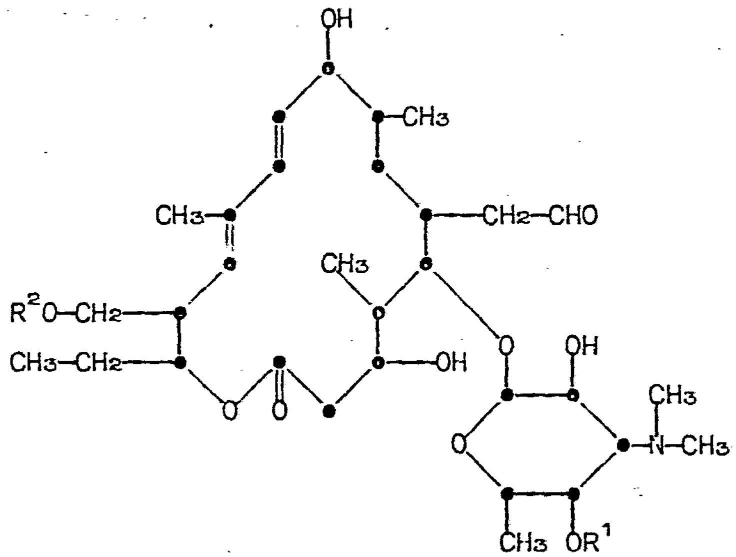

where $R^1$ and $R^2$ are as defined above, and in which the 20-aldehyde and free hydroxyl groups susceptible to acylation have been protected, followed by removal of the groups used to protect said aldehyde and hydroxyl groups.

2. A process according to claim 1 for preparing 9-O-acetyl-9-dihydrotylosin.

3. A process according to claim 1 for preparing 9-O-propionyl-9-dihydrotylosin.

4. A process according to claim 1 for preparing 9-O-benzoyl-9-dihydrotylosin.

5. A process according to claim 1 for preparing 9-O-acetyl-9-dihydrodemycarosyltylosin.

6. A process according to claim 1 for preparing 9-O-propionyl-9-dihydrodemycarosyltylosin.